(19) Europäisches Patentamt / European Patent Office / Office européen des brevets

(11) **EP 3 250 181 B1**

(12) **EUROPEAN PATENT SPECIFICATION**

(45) Date of publication and mention of the grant of the patent:
**03.06.2020 Bulletin 2020/23**

(21) Application number: **15798554.0**

(22) Date of filing: **08.10.2015**

(51) Int Cl.:
*A61K 9/00* (2006.01)          *A61K 9/06* (2006.01)
*A61K 47/36* (2006.01)          *A61K 47/40* (2006.01)
*A61K 47/50* (2017.01)

(86) International application number:
**PCT/IB2015/057694**

(87) International publication number:
**WO 2016/055961 (14.04.2016 Gazette 2016/15)**

(54) **MINOXIDIL-BASED PHARMACEUTICAL FORMULATION FOR THE TOPICAL USE AND KIT THEREOF**

AUF MINOXIDIL BASIERENDE PHARMAZEUTISCHE FORMULIERUNG ZUR TOPISCHEN ANWENDUNG UND KIT DAFÜR

FORMULATION PHARMACEUTIQUE À BASE DE MINOXIDIL POUR USAGE TOPIQUE ET TROUSSE CORRESPONDANTE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.10.2014 IT MI20141770**

(43) Date of publication of application:
**06.12.2017 Bulletin 2017/49**

(73) Proprietor: **Farmalabor S.r.l.**
**76012 Canosa Di Puglia (bt) (IT)**

(72) Inventors:
• **FONTANA, Sergio**
**76012 Canosa Di Puglia (BT) (IT)**
• **LA FORGIA, Flavia**
**70056 Molfetta (BA) (IT)**
• **LOPEDOTA, Angela Assunta**
**75100 Matera (IT)**
• **DENORA, Nunzio**
**70014 Conversano (BA) (IT)**
• **CUTRIGNELLI, Annalisa**
**70132 Bari (IT)**
• **FRANCO, Massimo**
**70126 Bari (IT)**
• **LAQUINTANA, Valentino**
**70043 Monopoli (BA) (IT)**

(74) Representative: **Colombo, Stefano Paolo et al**
**Marchi & Partners S.r.l.**
**Via Vittor Pisani, 13**
**20124 Milano (IT)**

(56) References cited:
**WO-A1-95/25500          US-A- 5 834 014**
**US-A1- 2011 112 125**

**Description**

The present invention refers to a minoxidil based pharmaceutical formulation for topical use (gel or viscose solution), relative use for the treatment of alopecia and kit for the preparation thereof. Minoxidil (MXD) is a powerful vasodilator which is prescribed for topical use in the treatment of alopecia of different origin.

This active ingredient is broadly used, both in male and female subjects, because it is able to reverse the progressive miniaturization of hair follicle associated to the androgenic alopecia directly acting on the proliferation and apoptosis of dermal papillae. Particularly, it is capable to act on scalp blood vessels promoting a greater oxygenation and nutrient supply to the hair follicle (see US 4,596,812; US 4,139,619).

For about twenty years, minoxidil has been prescribed for this therapeutic indication by dermatologists, as an industrial preparation or, frequently, as galenical.

However, minoxidil has multiple mechanisms of action responsible of both topical therapeutic effects and topical/systemic side effects. In fact, the main target of this molecule are ATP-dependent $K^+$ channels of the cardiac and muscular blood vessels made of the subunits SUR2A/B and Kir6.2. The activation of these channels induce a local vasodilatation of the cutaneous microcirculation with effects on hair trophism and on cell proliferation.

A severe minoxidil-mediated vasodilatation may cause acute headache observed during systemic use and topical use in the prolonged treatment lasting >20 days or in case of overdose.

The available minoxidil-based formulations in hydroalcoholic solution for topical use are characterized by poor handling with application problems, irritating and pro-inflammatory effects of the excipients.

Topical minoxidil-based formulations illustrated in Pharmacopea (Brusa P. et al. Prontuario pratico di galenica) or commercially available (Rogaine®/Regaine®) are generally solutions requiring the use of active ingredient concentrations ranging between 0.5% and 5% w/w. However, minoxidil has a low solubility in water (about 2.5 mg/ml), therefore the existing formulations on the market, usually, contain high percentage of cosolvents such as ethanol and propylene glycol.

For example, in order to achieve the solubilization of 5% w/w of minoxidil extra-strong Rogaine® solution or lotion for the scalp (Brusa P. et al.) should contain between 30 and 48% of ethanol and between 35 and 50% of propylene glycol. However, such solvents may cause different side effects, such as irritation, burning, itching, flushing and allergic contact dermatitis (Messenger AG et al., 2004; Tata S. et al., 1994; Tata S. et al., 1995; Edward S. et al., 2002). Additionally, ethanol easily evaporating after the application, may leave an oily residue on the scalp due to the presence of the propylene glycol, and may cause the precipitation of minoxidil crystals subtracting them to the skin uptake, thus reducing their bioavailability (Balakrishnan P. et al., 2002).

The international patent application WO95/25500 discloses formulations containing minoxidil and cyclodextrins in the presence of alcohol (ethanol), that is the fundamental strategy (cosolvency) for the solubilization of the active ingredient. Nevertheless, ethanol causes dryness of the treated area, and due to its quick evaporation results in a precipitation of minoxidil on the scalp, which is no more in solution and thus not available for the absorption by the stratum corneum to reach the hair follicle where it would act.

In the patent application US 2011/0112125 minoxidil solubility was improved thanks to the use of hydrophilic polymers such as carbomer, polyvinyl alcohol, polyacrylic acids, natural cyclodextrins etc., but the preparation of the formulation implies the use of 5M $H_2SO_4$. Minoxidil solubilization reached in the formulations according to US 2011/0112125 is due to the transformation of minoxidil into its salt form (sulfate) which is more soluble than its corresponding base and not to the presence of hydrophilic polymers added to the preparation. These polymers, including the β-CD, when used at low percentages, enable a limited solubilization of the active principle (see Example 10 below).

Therefore, attempts to enhance the aqueous solubility of minoxidil without the aid of cosolvents thus avoiding the formation of precipitates and to improve the bioavailability of minoxidil, become important challenges in the dermatological-pharmaceutical field. It is also of the outmost importance to identify novel minoxidil based formulations in order to reduce the side effects and to enhance the handling and the compliance of the product.

In order to ameliorate the drug skin penetration, several solutions have been proposed in the last years in alternative to the above reported traditional formulations, such as the innovative formulations constituted by microparticles, ethosomes, niosomes, liposomes etc. However, such pharmaceutical formulations, are difficult to be prepared, especially as far as galenical preparation is concerned, and do not enable always a drug solubilization in high amounts (Mura S. et al. 2009; Mura S. et al. 2011; Zhao Y. et al. 2010; Mura S. et al. 2007; EP 0177223).

In the patents US 5,030,442 and US 4,828,837 solubility enhancement of minoxidil was achieved by inclusion of amphiphatic excipients with a pKa less than 5. The use of such agents such as N-methyl cocoil taurate, lauryl sulphosuccinate, lysophosphatidic acid, monoalkyl phosphate ester, monoalkyl phosphonate, monoalkyl sulphonate, and oleamido-PEG-2-sulfosuccinate, may cause allergy problems.

Alternatively, solubilizing agents such as cyclodextrins have been also proposed.

It is well known that such cyclic oligosaccharides may form inclusion complexes with many hydrophobic drugs thus enhancing their water solubility (Stella V.J. et al., 1997). Such excipients have a limited toxicity and are in general considered safe.

There are several studies in the literature on the complexes between minoxidil and cyclodextrins (Calderini A. et al., 2008; Kim J. et al. 2003; Teak Kwan Kwon et al., 2010; Lopedota et al., 2014).

In the study authored by Calderini A. et al., 2008, minoxidil solubility has been tested through the use of β-CD resulting in the formation of an inclusion complex. However, such increase was not so high as expected in view of the inclusion constant ($K_c$ 150 M$^{-1}$).

Another limit of the β-CD is its low water solubility (1.8% w/v) when compared with its derivatives hydroxypropyl-beta cyclodextrin (HP-β-CD), sulfobutyl ether beta-cyclodextrin (SBE-β-CD), methyl-beta cyclodextrin (Me-β-CD), whose water solubility may also exceed 50 % w/v. Thus, β-CD may be used in very limited doses, not always sufficient to enhance the solubility of substances having low water solubility.

In the work authored by Kim J. et al. (2003), it is demonstrated that a solution of minoxidil and HP-β-CD has a reduced up-take in comparision to corresponding solutions containing cationic vescicles incorporating minoxidil.

On the contrary, Lopedota A. et al., 2014 disclose a Me-β-CD/minoxidil solution as alternative to HP-β-CD reporting minoxidil water solubility values and complexing capabilities similar to the solution with HP-β-CD. However, methyl cyclodextrins act by extracting cholesterol and triglycerides, reducing the skin barrier function and favouring an enhanced drug absorption (Irie T. and Uekama K., 1997). If on the one hand this may seem an advantage because such substances may behave as absorption enhancers, at the same time the reduction of the barrier function of the skin by extraction of cholesterol and triglycerides may cause mid and long term irritative effects.

The authors of the present invention have now prepared pharmaceutical formulations like viscose solution or gel comprising minoxidil and hydroxypropyl β-cyclodextrins as solubilizing agents for the topical treatment of alopecia, showing reduced irritative effcts (due to a limited specificity of the hydroxypropyl β-cyclodextrins towards cholesterol and triglycerids) wherein minoxidil is more effectively solubilized (see Example 10).

Such formulations conjugate the solubilizing effects of HP-β-CD against minoxidil, with the capability of better adherence and persistence of the gel formulations or viscose solutions on the scalp. The presence of hydroxypropyl β-cyclodextrin in due amounts and substitution degree allows drug solubilization as well with doses of 6% w/w.

The minoxidil solubilization principle adopted in the formulations according to the invention is based on a different strategy with respect to the cosolvency employed in the state of the art, that is the complexation. This is achieved by using a particular cyclodextrin (HP-β-CD), preferably with a substitution degree allowing to realize an inclusion complex with minoxidil, solubilizing up to 6% w/w of the latter. This new approach accomplishes a completely aqueous formulation enabling to overcome the above mentioned drawbacks relating to the use of both propylene glycol and ethanol (international patent application WO95/25500).

The gel formulations or viscose solutions according to the invention contain water as the vehicle and do not contain excipients like ethanol and propylene glycol responsible for the irritations of the scalp.

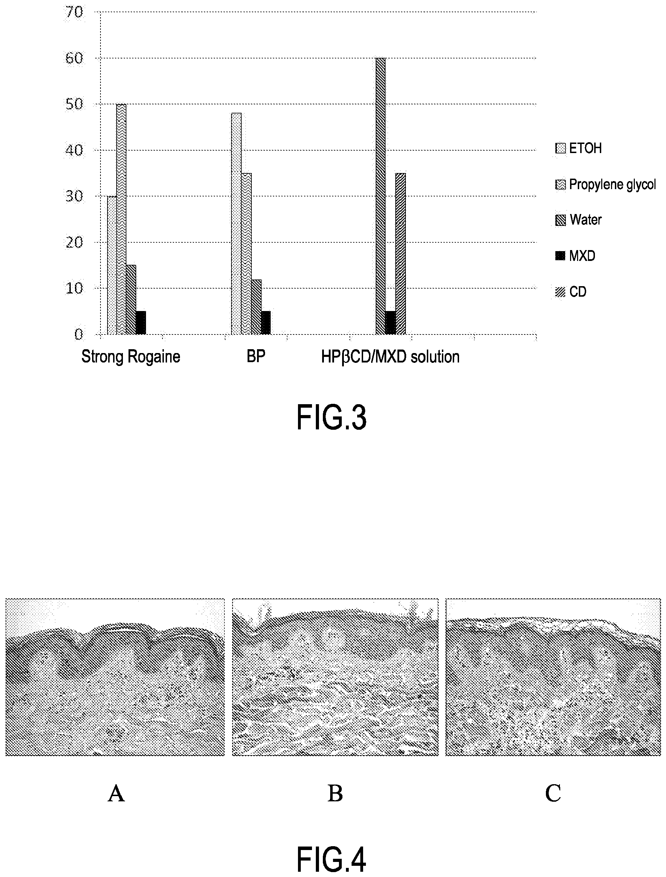

Figure 3 shows the comparison between the components of two commercially available preparations (MXD based topical formulations illustrated in Pharmacopoeia - Brusa P. et al. Prontuario pratico di galenica and Rogaine®) and one of the formulations herewith exemplified (Formulation A, Example 1), clearly showing the absence of the cosolvents ethanol/propylene glycol responsible for several side effects on the scalp.

Therefore it is the object of the present invention a gel formulation or viscose solution for topical treatment of alopecia comprising:

- minoxidil as active principle in a concentration comprised between 0.5% and 6% w/w;
- hydroxypropyl β-cyclodextrin as solubilizing agent in a concentration comprised between 5% and 45% w/w, wherein the substitution degree of the hydroxypropyl β-cyclodextrin is 4.5;
- at least an hydrophilic polymer selected among alginate, carbomer, polyacrylate, cellulose derivate, gelatin and pectin in a concentration comprised between 0.2% and 3% w/w;
- water between 42% and 85% w/w;

optionally together with one or more pharmaceutically acceptable excipients and/or adjuvants, characterized by the absence of the cosolvents ethanol and propylene glycol.

The formulations according to the invention depending on the selected hydrophilic polymer (alginate, carbomer, polyacrylate, cellulose derivate, gelatin and pectin), are easily obtained by hydration of the polymer in water (i.e. alginate, gelatin, pectin, cellulose derivate) or by addition of gelling agent such as inorganic and organic bases (i.e. carbomer, polyacrylate) or chlorides containing bivalent ions (i.e alginate).

Thus, according to an alternative embodiment, the formulation or viscose solution according to the invention further comprises at least a gelling agent selected between inorganic and organic strong bases (such as, for example, NaOH, KOH, BaOH$_2$, CaOH$_2$, triethanolamine) or chlorides containing bivalent ions (such as, for example, CaCl$_2$, BaCl$_2$, MgCl$_2$), at a concentration comprised between 2.6% and 4 % w/w.

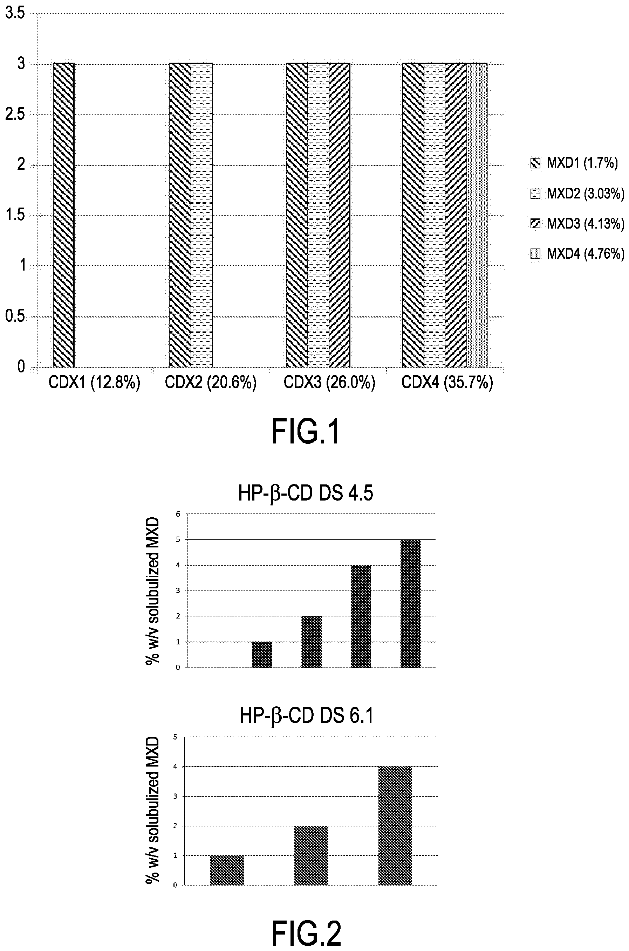

Preferably, hydrophilic polymers are present in a percentage comprised between 0.2 and 2%. Particularly, alginate gels (i.e. sodium or calcium alginate) were particularly advantegeous, as they show additional beneficial effects on the scalp injured by some forms of alopecia. Hydroxypropyl β-cyclodextrin has a substitution degree of 4.5. In fact, the authors of the invention identified that HP-β-CD with a substitution degree of 4.5 has a greater solubilizing activity than the same HP-β-CD with a substitution degree of 6.1 (see Example 4, Figure 2).

According to an alternative embodiment of the present invention, the gel formulation or viscose solution may contain hydroxypropyl β-cyclodextrin in combination with another β-cyclodextrin derivative selected between sulfobutyl ether beta-cyclodextrin (SBE-β-CD) and methyl β-cyclodextrin (Me-β-CD) with variable substitution degree, in a percentage comprised between 5% and 45% w/w.

Further excipients optionally present in the formulations according to the invention which may enhance the solubilizing effect of hydroxypropyl β-cyclodextrin are selected from the group consisting in hydrophilic excipients such as, for example, polyoxyethyleneglycol (PEG between 400 and 6000), polyvinylalcohol, polyvinyl pirrolidone, sorbitol, glycerine, lactose, saccharose, mannitol and are present in concentrations comprised between 0.2% and 5%. To this mixture is possible to add polymers that may enhance the solubilizing effect of hydroxypropyl β-cyclodextrin and/or absorption enhancers. Formulations according to the invention may further contain preserving agent such as antimicrobial and antifungal agents.

The present invention will be disclosed in the following for illustrative and not limitative purposes, on the basis of the appended figures, wherein:

- Figure 1 shows the effect on the solubilizing capacity of HP-β-CD with substitution degree 4.5 on four MXD percentages (1.7, 3.03, 4.13 and 4.76% w/w);
- Figure 2 shows the comparison between the solubilizing capacity of two types of HP-β-CD with different substitution degree 4.5 and 6.1;
- Figure 3 shows the comparison between the components of two commercially available preparations (BP and Rogaine®) and one of the formulations according to the invention (Formulation B), highlighting the absence of the cosolvents ethanol/propylene glycol responsible for different side effects on the scalp;
- Figure 4 illustrates the results of the histological examination on pig ear skin: A) control; B) skin treated with an aqueous solution of 33% w/w HP-β-CD, and minoxidil 5% w/w for 24 hrs at 37°C; C) skin treated with the solution prepared following the indications of the BP (48% ethanol, 35% propylene glycol, 12% water and 5% minoxidil) for 24 hrs at 37°C.
- Figure 5 shows the pictures of the three depilation experimental groups on day 0 of treatment.
- Figure 6 shows the three experimental groups on the first week of treatment: group I CTRL, group II commercial solution, group III formulation A.
- Figure 7 shows the three experimental groups on the second week of treatment: group I CTRL, group II commercial solution, group III formulation A.
- Figure 8 shows the three experimental groups on the third week of treatment: group I CTRL, group II commercial solution, group III formulation A.
- Figure 9 shows the slides of the rat hair from the three experimental groups: group I CTRL, group II commercial solution, group III formulation A.
- Figure 10 shows a graph which reports the hair growth trend in the rats under treatment.
- Figure 11 shows two graphs illustrating the hair lenght/diameter growth trend in the rats under treatment.
- Figure 12 shows the gene expression of the autophagy marker Bnip3 (panel A) and of the atrophy marker Atrogin-1 (panel B) both in the skin and the heart.
- Figure 13 shows the gene expression of the caspasi 3 marker (panel A) and of the marker Mapk3 (panel B).
- Figure 14 shows the gene expression of the markers Nfkb1 and TNFα (panels A and B).
- Figure 15 shows the phase solubility graph of MXD/HPβCD at 25°C.

EXAMPLE 1: *Exemplifying gel formulations of the invention*

Formulation A

|  |  |
| --- | --- |
| - Mixture A: |  |
| ✔ HP-β-CD (substitution degree 4.5) | 23.16 w/w |
| ✔ sodium alginate | 0.5 w/w |
| - Minoxidil | 3.51 w/w |

(continued)

- Solution B:
  ✓ calcium chloride dihydrate solution    0.1 M
                   2.63 w/w
  ✓ preserved water            70.2 w/w

Preparation:

The powder mixture made of HP-$\beta$-CD and sodium alginate was solubilized in water, followed by addition of minoxidil base, which solubilized under continuous magnetic stirring. The product required about 1 hour for a complete solubilization. It is recommended to adjust the magnet speed to the minimum in order to avoid air bubble incorporation. The solubilized product was clear, colourless and viscose. Once solubilization is completed a 0.1 M $CaCl_2$ solution was added dropwise under sustained magnetic stirring. Gel required from 8 to 12 hrs to complete its gelification.

Formulation B

| | |
|---|---|
| - HP-$\beta$-CD (substitution degree 4.5) | 23.24 w/w |
| - Sodium Alginate | 1.41 w/w |
| - Minoxidil | 3.52 w/w |
| - Preserved water | 71.83 w/w |

Preparation:

To the powder mixture made of HP-$\beta$-CD and sodium alginate, minoxidil base powder was added.

Said mixture was transferred to a becker wherein purified water was added slowly. Powders were left solubilizing under continuous magnetic stirring. The product required about 1 hr for a complete solubilization. It is recommended to adjust the magnet speed to the minimum in order to avoid air bubble incorporation. After 5 hrs the product appears like a gel.

Formulation C

| | |
|---|---|
| - HP-$\beta$-CD (substitution degree 4.5) | 23.75 w/w |
| - Carbomer 934 | 0.36 w/w |
| - NaOH solution (0.1 M) | 0.36 w/w |
| - minoxidil | 3.59 w/w |
| - preserved water | 71.83 w/w |

Preparation:

To the powder mixture made of HP-$\beta$-CD and carbomer, minoxidil was added. Said mixture was transferred to a becker wherein purified water was added slowly. Powders were left solubilizing under continuous magnetic stirring. The product required about 3 hrs for a complete solubilization. It is recommended to adjust the magnet speed to the minimum in order to avoid air bubble incorporation. 0.1 M NaOH solution was added dropwise to the viscose solution until a gel is obtained.

Formulation D

| | |
|---|---|
| - HP-$\beta$-CD (substitution degree 4.5) | 24.0 w/w |
| - Hydroxyethylcellulose (HEC) | 1.0 w/w |

(continued)

| | |
|---|---|
| - Minoxidil | 3.52 w/w |
| - Preserved water | 71.48 w/w |

To the powder mixture made of HP-β-CD and hydroxyethylcellulose, minoxidil was added. Said mixture was transferred to a becker wherein purified water was added slowly. Powders were left solubilizing under continuous magnetic stirring. The product required about 1 hr for a complete solubilization. It is recommended to adjust the magnet speed to the minimum in order to avoid air bubble incorporation. After 5 hrs the product appears like a gel.

Formulation E

| | |
|---|---|
| - Mixture A: | |
| ✓ HP-β-CD (substitution degree 4.5 or 6.1) | 18 w/w |
| ✓ sodium alginate | 0.5 w/w |
| - Minoxidil | 2.0 w/w |
| - Solution B: | |
| ✓ calcium chloride dihydrate solution | 0.1 M |
| | 2.7 w/w |
| ✓ preserved water | 76.8 w/w |

Preparation:

The powder mixture made of HP-β-CD and sodium alginate was solubilized in water, followed by addition of minoxidil base, which solubilized under continuous magnetic stirring. The product required about 1 hour for a complete solubilization. It is recommended to adjust the magnet speed to the minimum in order to avoid air bubble incorporation. The solubilized product was clear, colourless and viscose. Once solubilization is completed a 0.1 M $CaCl_2$ solution was added dropwise under sustained magnetic stirring. Gel required from 8 to 12 hrs to complete its gelification.

Formulation F

| | |
|---|---|
| - Mixture A: | |
| ✓ HP-β-CD (substitution degree 4.5) | 40.0 w/w |
| ✓ sodium alginate | 0.5 w/w |
| - Minoxidil | 6.0 w/w |
| - Mixture B: | |
| ✓ calcium chloride dihydrate solution | 0.1 M |
| | 3.0 w/w |
| ✓ preserved water | 45.5 w/w |

Preparation:

The powder mixture made of HP-β-CD and sodium alginate was solubilized in water, followed by addition of minoxidil. The components were solubilized after about 1 hour at room temperature and under continuous magnetic stirring. It is recommended to adjust the magnet speed to the minimum in order to avoid air bubble incorporation. The solubilized product was clear, colourless and viscose. Once solubilization is completed a 0.1 M $CaCl_2$ solution was added dropwise under sustained magnetic stirring. Gel required from 8 to 12 hrs to complete its gelification.

Formulation G

- Mixture A:
  - ✓ HP-β-CD (substitution degree 4.5)    24 w/w
  - ✓ sodium alginate    0.5 w/w
- Minoxidil    3.51 w/w
- Solution B:
  - ✓ capryl-caproyl macrogol 8-glyceride    2 w/w
  - ✓ preserved water    67.29 w/w
- Solution C:
- calcium chloride dihydrate solution    0.1 M
    2.7 w/w

Preparation:

The powder mixture made of HP-β-CD and sodium alginate was solubilized in the solution B followed by addition of minoxidil base which solubilized under continuous magnetic stirring. The product required about 1 hour for a complete solubilization. It is recommended to adjust the magnet speed to the minimum in order to avoid air bubble incorporation. The solubilized product was clear, colourless and viscose. Once solubilization is completed the solution C was added dropwise under sustained magnetic stirring. Gel required from 8 to 12 hrs to complete its gelification.

Formulation H

- Mixture A:
  - ✓ HP-β-CD (substitution degree 4.5 or 6.1)    24 w/w
  - ✓ sodium alginate    0.5 w/w
  - ✓ PEG 6000    1.0 w/w
- Minoxidil    4.0 w/w
- Solution B
  - ✓ capryl-caproyl macrogol 8-glyceride    2 w/w
  - ✓ preserved water    65.8 w/w
- Solution C:
  - ✓ calcium chloride dihydrate solution    0.1 M
    2.7 w/w

Preparation:

The powder mixture made of HP-β-CD and sodium alginate and PEG 6000 was solubilized in the solution B followed by addition of minoxidil. After 3 hrs at room temperature and under continuous magnetic stirring, the preparation is clear, colourless and viscose. Then 0.1 M $CaCl_2$ solution was added dropwise under sustained magnetic stirring. Gel required from 8 to 12 hrs to complete its gelification.

EXAMPLE 2: *Analysis of the organoleptic characteristics of the formulations A-D according to the invention*

The organoleptic characteristics of some of the gel formulations of the invention (Formulations A-D, Example 1) were analyzed.

Homogeneity (aggregates presence) was evaluated by visual inspection after inserting the formulation into suitable vials; the presence of solid particles was detected by optical microscope (Leica Galen III equipped with Panasonic camera and Leica Qwin software 2.4).

pH was measured by a pH-meter (Inolab pH LEVEL 1 WTW, Weilheim, Germany). 50 mg of the specific gel were dissolved in 50 ml of distilled water for 2 hrs at room temperature. pH of this solution was measured.

The following Table 1 illustrates the achieved results.

Table 1: Organoleptic characteristics of some of the gel formulations of the invention (Formulations A-D)

| Gel | Formulation | Homogeneity | Grittiness | pH |
|---|---|---|---|---|
| Calcium alginate | A | +++ | - | 6.52±0.5 |
| Sodium alginate | B | +++ | - | 6.50±0.2 |
| Carbopol 934 | C | ++ | - | 6.53±0.8 |
| HEC | D | +++ | - | 6.50±0.5 |
| Excellent +++, Good ++, Satisfying+, No grittiness - | | | | |

EXAMPLE 3: *Study on the effect of the solubilizing capacity of the HP-β-CD with substitution degree 4.5*

Preliminary studies were carried out in order to establish: a) which HP-β-CD substitution degree is better to be used in the formulations and b) the quantitative range necessary to solubilize minoxidil in the amount fixed in the range between 0.5%-6% w/w.

MATERIALS AND METHODS

Preparations containing HP-β-CD with substitution degree 4.5 in different concentrations (15, 27, 39 and 60 g in q.s. to 100 ml of water) and minoxidil in different concentrations (2, 4, 6 and 8 % w/v) were assayed.
In detail, four solutions were set up containing: 15 g, 27 g, 39 g and 60 g of HP- β-CD in 100 g of water. 2 grams of minoxidil were added to the first solution, 4 g to the second; 6 g to the third; and 8 g to the fourth.
The preparations were left under magnetic stirring for one hour and then the active principle solubilization was evaluated by visual inspection (limpidity of the preparation).
Solubility data were collected three times for each formulation.

TESTED FORMULATIONS

1) First formulation containing 15 g of cyclodextrin and 2 g of minoxidil on 100 g of preparation;
2) Second formulation containing 27 g of cyclodextrin and 4 g of minoxidil on 100 g of preparation;
3) Third formulation containing 39 g of cyclodextrin and 4 g of minoxidil on 100 g of preparation;
4) Fourth formulation containing 60 g of cyclodextrin and 8 g of minoxidil on 100 g of preparation.

RESULTS

Figure 1 illustrates the HP-β-cyclodextrin values expressed in w/w relative to the first to fourth preparations being 12.8%, 20.6%, 26.0% and 35.7% w/w respectively, and the corresponding solubilized minoxidil values being 1.7%, 3.03%, 4.13% and 4.76% w/w.
At the HP-β-CD concentration of 12.8% w/w only 1.7% w/w of minoxidil may be solubilized; at the HP-β-CD concentration of 20.6% w/w both 1.7% w/w and 3.03% w/w of minoxidil is solubilized; at the HP-β-CD concentration of 26% w/w 1,7%, 3.03% and 4.13 % w/w of minoxidil is solubilized, finally at the concentration of 35.7% w/w it is possible to solubilize up to 4.76% w/w of minoxidil.
Figure 1 graphically illustrates the results obtained on the different solubilizing capacity of HP-β-CD with substitution degree 4.5.

EXAMPLE 4: *Comparison study on the solubilizing capacity of the HP-β-CD with different substitution degree*

MATERIALS AND METHODS

Two solutions of HP-β-CD at 24.8% w/w with two different cyclodextrins each having a substitution degree of 4.5 and 6.1, were prepared.
Particularly, 33 grams for each cyclodextrin were solubilized in 100 ml of water (or 24.8 g of CD and water q.s. at 100 g).

Said solution was distributed in vials in 5 ml volume aliquots. Each vial was added with an amount of minoxidil of 1% w/v (50 mg); if the active principle was dissolved (visual inspection) other 50 mg were added to obtain a minoxidil concentration of 2% w/v until an unclear solution with a suspended product is visually obtained.

TESTED FORMULATIONS

100 g of formulation containing 24.8 g of HP-β-CD with a substitution degree of 4.5 and 100 g of formulation containing 24.8 g of HP- β -CD with a substitution degree of 6.1.

RESULTS

Up to 5% w/v minoxidil solubilizing capacity in formulations comprising HP-β-CD in a concentration of 24.8% w/w (with a substitution degree 4.5 and 6.1) was tested.
The formulation comprising HP-β-CD with a substitution degree of 4.5 allows a suitable minoxidil solubilization up to 5%, whilst the formulation comprising HP-β-CD with a substitution degree 6.1 is not capable to solubilize such amount stopping at 4%.
Figure 2 shows graphically the results obtained on the different solubilizing capability of HP-β-CD with a substitution degree of 4.5 and 6.1, respectively.

EXAMPLE 5: *Comparison of the water, ethanol and propylene glycol content between one of the formulation according to the invention and two commercially available formulations*

MATERIALS AND METHODS

The amounts of solvents and cosolvents (water, propylene glycol and ethanol) employed to solubilize 5 g of minoxidil were identified from the literature (Brusa P. et al. and US 2011/0112125 A1).
Data refer to 100 g final product containing 5 g of minoxidil.
Such values were compared with a preparation according to the invention having the same amount of minoxidil.

FORMULATIONS

The following formulations containing 5% minoxidil were compared:

A) extra strong Rogaine: formulation containing 30% ethanol, 50% propylene glycol and 15% water
B) Formulation reported in the British Pharmacopea (BP) containing 48% ethanol, 35% propylene glycol and 12% water
C) Water formulation containing 33% w/w HP-β-CD, 62% water

RESULTS

From such comparison it is clear the absence of cosolvents in the formulation C and the fact that this preparation shows the same minoxidil solubilizing capability of the formulations A and B (see Figure 3).

EXAMPLE 6: *Study on the histological effect of the presence of ethanol and propylene glycol*

MATERIALS AND METHODS

In a Franz cell having a surface area of 2.009 cm$^2$ and a receiving volume of 12.3 ml, pig ears skin (full tickness) taken from sacrificed pigs in the abattoir were fixed.
The ears were rinsed under running water, depilated and the skin was removed with a bistoury by eliminating the muscular and fat portions. One part of the tissue as such (control) was suitably treated for the histological evaluations.
Other two portions were mounted on a Franz cell.
In the receiving compartment 1 ml of the minoxidil and cyclodextrin solution was obtained by solubilizing 5 g of the active principle in the presence of 33 g of HP-β-CD in water q.s. to 100 g. In the receiving compartment of another Franz cell, 1 ml of the prepared formulation were added according to BP instructions (Brusa P. et al.).
In detail, 5 g of minoxidil were solubilized under stirring in the mixture made by propylen glycole (35 g) and ethanol 96° (48 g), then water is added under stirring q.s. at 100 g (12 g)
The two Franz cells were thermostated at 37°C in order to reach a temperature of 32°C for 24 hours on the skin surface. At the end of this period the skin samples were treated for histological evaluation.

In particular, samples were fixed in formalin for 24 hours, dehydrated with raising concentrations of ethylic acid, embedded in paraffin and then dissected at microtome in slices of 2-5 micrometers. Such slices were paraffin deprived, hydrated with decreasing concentration of alcoholic solutions, finally stained with hemallume and eosin. Stained slides were observed under light microscope.

COMPARISON FORMULATIONS

Formulation containing 33 g of HP-β-CD and 5 g of minoxidil on 100 ml of water.

Formulation prepared according to the receipt reported in BP: Minoxidil 5 g; propylene glycol 35 g; ethanol 96°, water 48 g q.s. to 100 g.

RESULTS

Figure 4 shows the results of the histological examination carried out on the pig ears skin treated for 24 hours at 37°C, respectively with:

- control: skin structure without any treatment (panel A),
- formulation with cyclodextrin B according to the invention (panel B)
- solution prepared according to the BP instructions (Prontuario pratico di galenica authored by Brusa P.) (panel C).

The histological examination reported in panel B does not reveal any particular alteration of the stratum corneum; whilst the one reported in panel C shows a substantial disorganization of the stratum corneum.

EXAMPLE 7: *Study on the in vivo efficacy of the formulation A according to the invention*

The efficacy of one of the formulations according to the present patent application (formulation A; example 1) was evaluated *in vivo* on a group of male rats.

MATERIALS AND METHODS

*In vivo* studies on male rats to test the efficacy of the new formulation containing 3.5% w/w minoxidil were carried out. The results of such studies were compared with the commercial available minoxidil solution (3.5% w/w hydroalcoholic solution) and with the control (untreated rats). The study is divided in two experimental phases:

First pilot phase

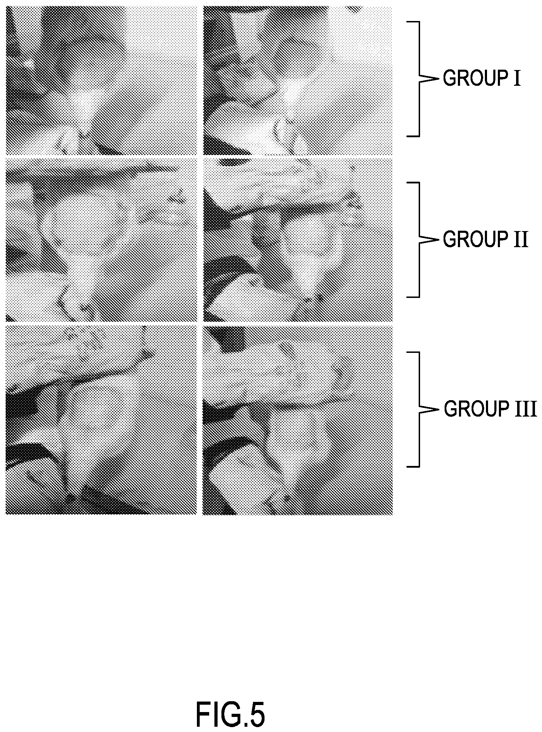

In a first phase a test to evaluate the efficacy of the formulation under development (formulation A) in comparison to the commercial available product was carried out. This study has been carried out on six 8-months old male rats (adult-old), average weight 550 g. Before and during the treatment period, rats were normally fed; they were shaved on their back by an electric razor the day before the starting of the treatment in order to obtain a shaven area of 10 cm2. The complete depilation of the area was achieved by applying a depilatory cream for a 3 min period. The cream was removed and the skin was treated with a 70% ethanol hydroalcoholic solution. Rats were randomly selected and splitted in three groups, each contemplating two rats. Figure 5 shows the pictures of the three depilation experimental groups at day 0 of treatment.

Groups under study:

Group I: not treated control group (CTRL) observed for a period of 23 days;
Group II: group treated with 1 ml di of the commercial available Minoxidil solution (3.5% w/w) topically applied once a day on the shaved part for a period of 23 days;
Group III: group treated with hydrogel containing 1 g of Minoxidil (3.5% w/w) applied in the same manner of Group II.

Rats from the three groups were observed and photographed in different days. At fixed days hair withdrawal for macro- and microscopic evaluation of diameter, length and appearance, as well as of bulb size was carried out.

Photographic analysis

Hair growth area was photographed weekly for each rat by iphone 5S and images were analysed by the Microsoft Office Picture Manager program.

Sampling and trichological analysis

All the following analysis were carried out under blind conditions. Hair withdrawal was carried out periodically and all the samples were analysed by light microscope (Leica Gallen III 50x, 100x, 400x magnification, equipped with Panasonic camera with Leica Qwin software, version 2.4), to evaluate the hair structure and the bulb size. Additionally, hair length on a statistically significant number of different samples was measured, through a caliber with millimeter precision.

RESULTS OF THE FIRST PILOT PHASE

During the 23 days-treatment the rats belonging to the three groups were observed and photographed periodically.

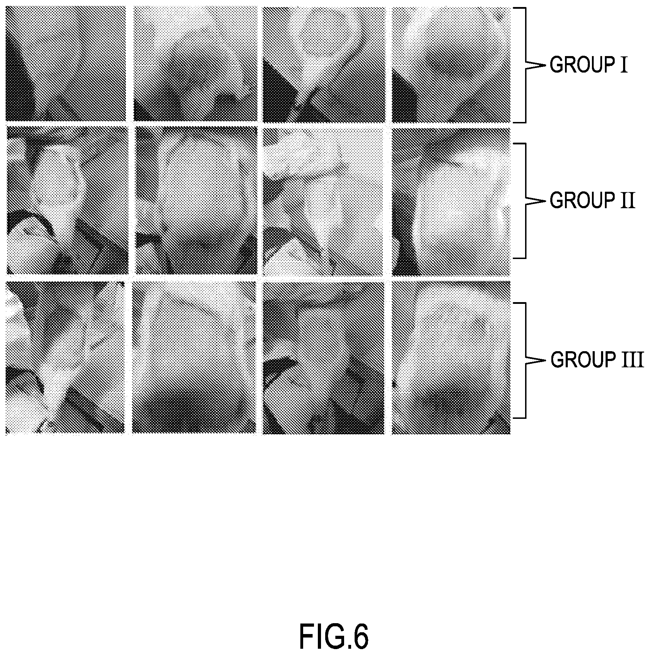

From daily observations and photographs taken periodically it is observed that the two treated groups with group II (commercial available solution) and group III (formulation A; example 1) showed a comparable hair growth, with a coverage shaved area of 20% in group II and 30% in group III since the beginning of the treatment (first week). On the contrary group I (control, CTRL) shows a very slow hair growth with a covered surface of 10% (Figure 6). Figure 6 shows the three experimental groups at the first week of treatment: group I CTRL, group II commercial available solution, group III formulation A.

By setting up the treatment threshold at 20% of the covered body surface on the first week of treatment, we may conclude that two rats from groups II and III responded to the treatment (responders): rat 1 from group II and rat 1 from group III.

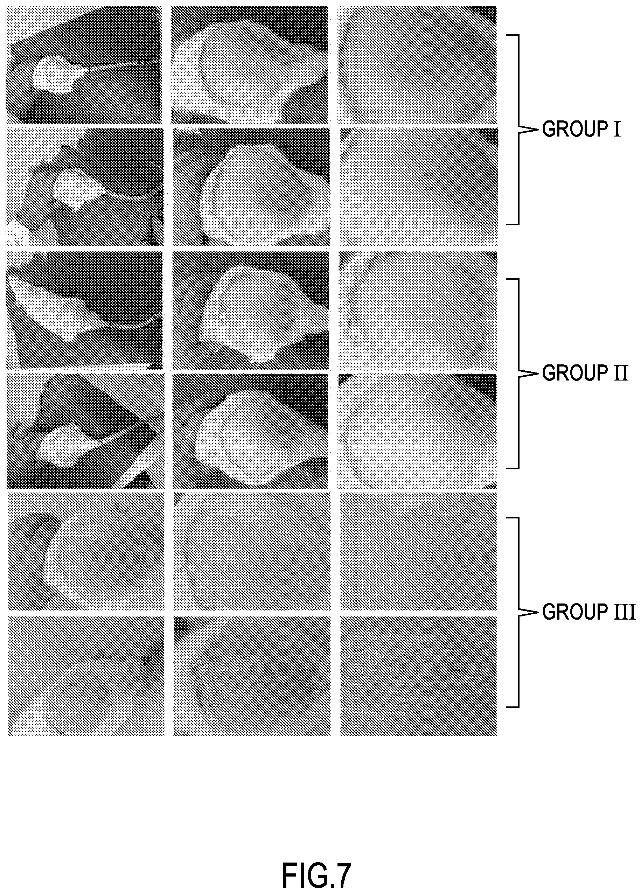

On the second week both rats from group III showed hair growth of, respectively, 60% and 55%, whilst in group II of 30% and 18% and no responder was observed in the control group I (hair growth percentage of 15% and 10%) (Figure 7).

It is possible to identify: two responder rats in group III, 1 responder rat in group II, no responder rat in group I.

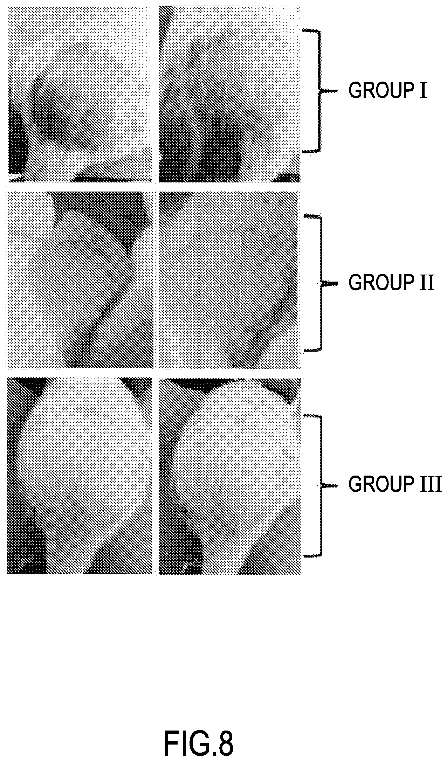

On the third week of treatment group III showed a complete coverage of the shaved area equal to 100% vs group I. Also group II shows 100% coverage, but with a greater hair thinning (Figure 8). Group I showed a coverage of the shaved skin area not greater than 60%.

Hair quality and length

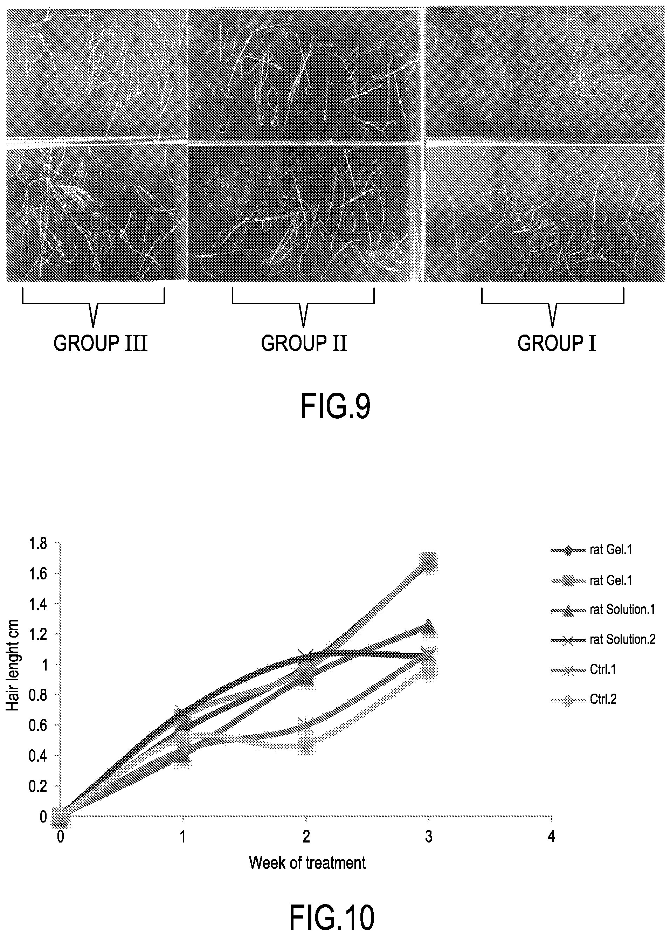

Hair quality was examined in all the experimental groups. In detail, samples from group III resulted thicker and longer than group II and such difference is significantly more remarkable when compared to group I (Figure 9).

Figure 10 shows a graph illustrating the hair growth trend in a 4 weeks observation period in the rats under treatment.

SECOND PHASE

In the light of the results of the first study, an in-depth program of *in vivo* experiments was carried out in order to confirm the observations made in the first study, and to contextually evaluate side and irritating effects of the formulation under analysis.

In this phase the study was carried out on nine 10-12 months old male rats (adult-old) and weighting 750 g on average, normally fed. The animals were shaved and depilated as above disclosed. Rats were randomly selected and splitted into three groups.

Study group:

Group I: not treated control group (CTRL) observed for the whole period;
Group II: group treated with 1 ml di of the commercial available Minoxidil solution (3.5% w/w) topically applied once a day on the shaved part for a period of 25 days.
For the period following the 25th day, the dose was applied twice a day (morning and evening) until the 40th day of

treatment.
Group III: group treated with 1 g of formulation A (3.5% w/w) applied in the same manner of Group II.

Rats from the three groups were observed and photographed in different days. At fixed days hair withdrawal for macro- and microscopic evaluation of diameter, length and appearance, as well as of bulb size was carried out.
On the treatment day 40, each animal was weighted, anaesthetized with urethane administered by intraperitoneal route at the concentration of 1.2 g/kg, and depilated on the treated back and being subjected to:

- blood sampling from left heart ventricle;
- biopsy withdrawal from dorsal skin to carry out inflammation marker gene expression studies, by RT-PCR technique on total mRNA tissue extracts;
- biopsy withdrawal from dorsal skin treated area for histological analysis evaluating:

  a. number and diameter of hair bulbs;
  b. inflammatory status and search for possible necrosis area;

- heart explant for the gene expression analysis of inflammation marker by RT-PCR technique on total mRNA tissue extracts;
- biopsy withdrawal fromm ventral skin for primary culture of fibroblasts.

All the animals completed the 40 days treatment protocol without clear clinical intolerance effect or cutaneous reactions, but those belonging to group II.
In this group one rat at the treatment day 25 was died probably for hypotensive shock (no post mortem examination was carried out).

RESULTS

*Growth rate and trichological examination*

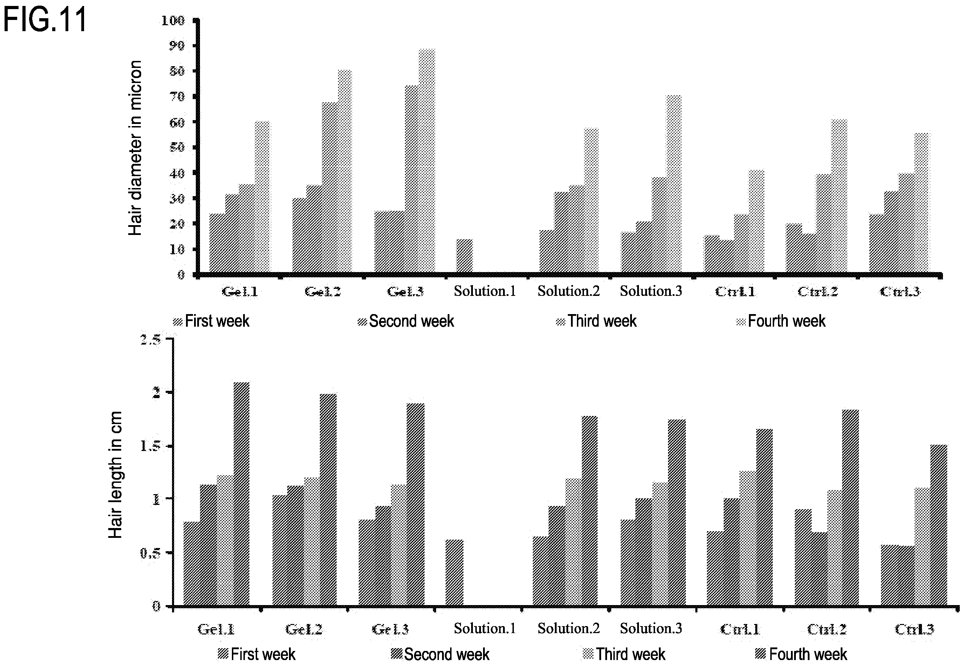

Daily monitoring and observations of the three groups confirmed the results of the first phase concerning the hair growth rate and the hair coverage of the treated area.
In fact, also in this study group III (Gel) showed a faster growth in comparison to groups II (solution) and I (Ctrl) especially after the first week, with a better hair quality in terms of diameter and length.
Group I showed a definitely lower growth with a thin and fragile hair quality.
Microscopic analysis of the periodically withdrawn hair showed a greater length and diameter of group III vs group II and group I (see graphics in Figure 11).

*Histological data*

Preliminary histological analysis of hair sections in formaldehyde showed a significant enhance of the bulb number for histological area in group III vs group II and group I, in agreement with the hair density increase observed in the growth rate and trichological examination studies.

*Gene expression and heart and dorsal skin inflammation*

Gene expression analysis carried out by RT-PCR technique from skin and heart tissue samples explanted from treated rat, showed:

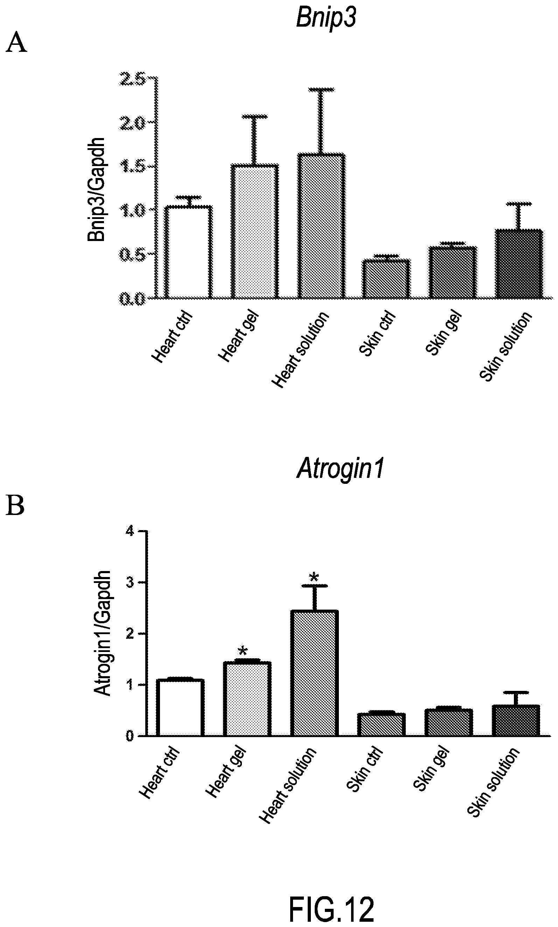

- no meaningful enhancement of the gene expression of the autophagy marker Bnip3 and a significant enhancement of the atrophy gene marker Atrogin-1 both in the skin and in the heart: group II > group III > group I (Figure 12, panels A and B).

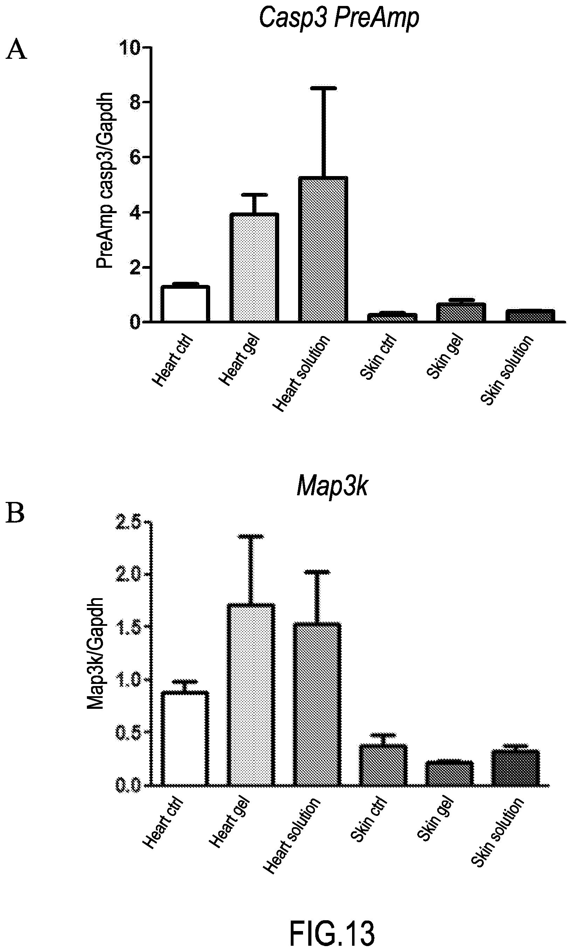

These results point out a greater autophagic and atrophic effect of the minoxidil based commercial solution vs minoxidil based formulation A both in the skin and in the heart.
Caspasi 3 gene, which is an apoptosis marker in different tissues, is significantly expressed in the heart of animals belonging to group II and group III:
Minoxidil based commercial solution $\geq$ minoxidil based gel formulation A > control thus suggesting a similar pro-apoptotic effect of the treatment for both the formulations (Figure 13, panel A).

As far as the gene expression of the Mapk3 marker is concerned, which is an enzyme involved in tissue hypertrophy, it was observed an enhancement of its expression in the heart both with the formulation A (gel) and with the commercial solution (Figure 13, panel B).

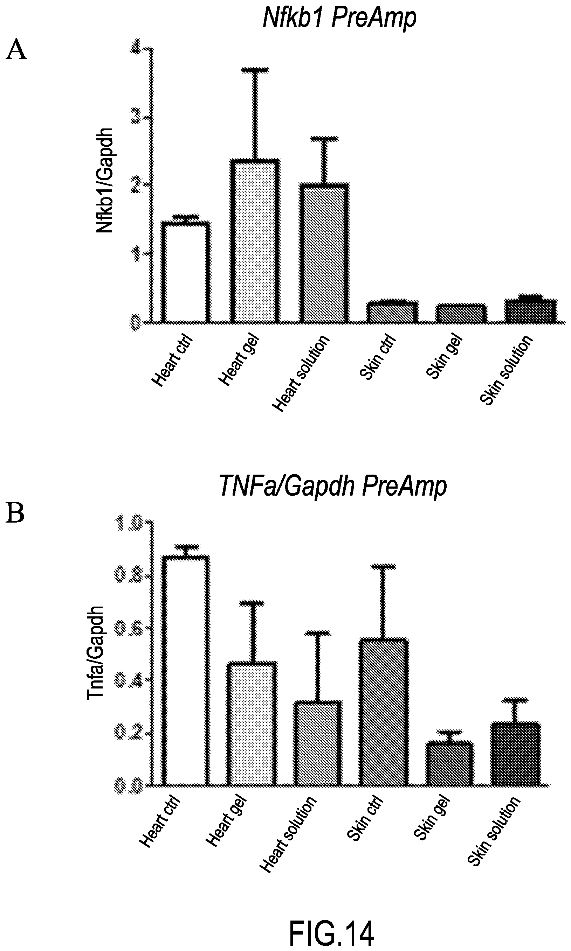

Nfkb1 and TNF$\alpha$ marker gene expression, does not appear to be relevant in this experimental groups thus suggesting that the cytokines pathway is not involved in the inflammatory processes (see Figure 14, panels A and B).

*Pressure measurements and ECG in wireless telemetry in rats treated topically with Minoxidil based GEL in GLP*

Preliminary longitudinal telemetric measurements (ADInstruments, UK; Millar New Zealand) taken on three rats, showed that at the effective dose the treatment induces a slight reduction of the aortic blood pressure in the rat treated with the formulation A (gel) and a moderate reduction in the rat treated with the commercial solution. Such results suggest that the long term treatment (40 days) may induce an hypotension in the two treatment groups, that is greater in the group treated with the Minoxidil based commercial solution.

The above reported preliminary pharmacological study carried out on male adult-old rats, showed the superiority of the new minoxidil based gel formulation in terms of efficacy, handling, reduced local and systemic toxicity.

EXAMPLE 8: *Phase solubility study: determination of the complexation constant between Minoxidil and HP-$\beta$-CD*

Complexation between Minoxidil and cyclodextrins was studied by different researchers and particularly Kim J. et al. (2003) and Kwon T. K. et al. (2010), carried out studies on phase solubility, through spectrophotometry between Minoxidil and HP-$\beta$-CD in the concentration range comprised between 0 and 0.32 M. However, in such works the substitution degree of the HP-$\beta$-CD is equal to 0.8 (Kim et al. 2003) or not disclosed (Kwon et al. 2010).

HP-$\beta$-CD substitution degree, is a key parameter to be considered because it could be a discriminating factor in order to determine the Minoxidil solubility enhancement as shown in preceding Examples 3-4.

On the basis of the above reported considerations solubility constant between Minoxidil and HP-$\beta$-CD with substitution degree 0.64 (suggested as the most preferred embodiment of the present invention) was evaluated.

Among the possible approaches used to calculate the solubility constant, in the present study the solubility method introduced by Higuchi and Connors (1965) was adopted.

MATERIALS AND METHODS

For the phase solubility study an amount of Minoxidil exceeding its solubility was added to 5 non-buffered aqueous solutions having increasing concentration of HP-$\beta$-CD (range 0-0.128 M) in 5 ml sealed vials, to avoid any change caused by evaporation.

Suspensions thus obtained were stirred by vortexing for about 5 minutes, and subsequently transferred in a orbital shaker in the dark for 2 days at 25 $\pm$ 0.2°C at 100 oscillations per minute (Themo Scientific).

Once equilibrium is reached, an aliquot of the aqueous phase of each sample has been transferred to a 5 ml glass syringe and filtered through a cellulose acetate membrane filter of 0.22-$\mu$m (Millipore®, Milan, Italy).

Filtrate was stored in the dark at temperature of 25 $\pm$ 0.2°C until the next analysis which was carried out in HPLC after opportune dilution of the sample. All the measurements were repeated at least in triplicate.

RESULTS

Water solubility (S0) of Minoxidil was determined in the same way and resulted consistent with the literature data (2.5 mg/ml).

Data retrieved from the quantitative analysis of Minoxidil via HPLC were used for determining the inclusion complex constant MXD/HP-$\beta$-CD, according to the Higuchi-Connors equation, reported in the following:

$$K_{(1:1)} = \text{Slope}/(S_0 \, (1\text{-Slope}))$$

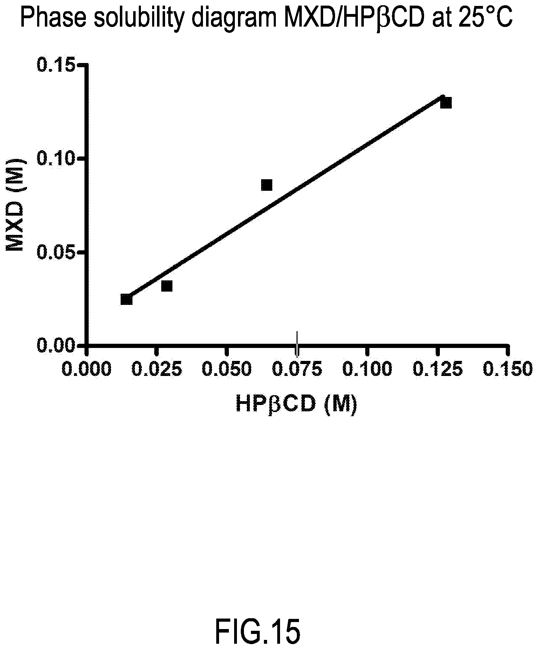

From the results of the study it is possible to state that, in the envisaged concentration range, the HP-$\beta$-CD/minoxidil complex is formed in a molar ratio 1:1 CD:MXD (see Figure 15).

The complexation constant calculated results to be equal to 1500 M$^{-1}$, such value being definitively higher than the one indicated by Kim et al. Such revealed difference may be associated to the analytical method employed in the study (HPLC vs UV), and above all to the different substitution degree of the cyclodextrin selected for these two studies, thus confirming the importance of the selection of the substitution degree of the HP-$\beta$-CD.

EXAMPLE 9: *Stability studies of the formulations according to the invention*

The formulation stability is fundamental in order to establish its expiry date. It is necessary to analyze the preparation under various aspects in order to evaluate stability: chemical, physical and microbiological aspects. The minoxidil based master formulations reported in Pharmacopoeia, having alcohol percentage greater than 25% w/w, resulted stable from a microbiological point of view and it is possible to indicate an expiry date of 6 months from the date of preparation. However, it is advisable the storage in dark glass bottles for the issue of poor photostability of Minoxidil.

In order to establish a possible expiry date of the formulation object of the invention, three chemical-physical stability studies of the formulation and of the active principle were conducted for a maximum time period of three months.

The first study was carried out under real storage conditions, for three months and a total of 35 days. In the second study the same formulation was stressed under a cold-warm cycle in a climate room for a cycle of total 60 hours. In the third study the gel formulation prepared using preserved water was put in the climate room for 3 months at 25 $\pm$0.2°C and 60% RH.

Methods

In the first study, a formulation A was prepared according to the present invention. In detail 10 grams of Minoxidil gel 3.5% w/w were placed in transparent containers covered with parafilm and left on a shelf at room temperature either protected or not from the light. Each week a gel aliquot was taken from the container and diluted with an appropriate amount of water/methanol. One part of the remaining solution was filtered and injected in HPLC (20 $\mu$l) in order to evaluate the amount of Minoxidil which is present.

The integrated area relative to the chromatographic peak of Minoxidil was transformed in Minoxidil concentration (mg/ml) based on a previously prepared calibration curve (R2 0.998). The obtained value was transformed in a percentage value with respect to the expected value (0.035 mg/ml) representing the amount of Minoxidil corresponding to 100% of said active into the gel.

In the second study, chemical and physical stability of Minoxidil in the same formulation was evaluated in climate room (Climacell, MMM Medcenter-Munich, Germany) for 60 hours adopting the following programs:

- first program in the dark and 5 sections; 10 grams of Minoxidil gel 3.5% w/w (formulation A) in a transparent glass container for 12 hours at 4°C; 12 hours at 25°C and 60% of R.H.; 12 hours at 40°C and 60% R.H.; 12 hours at 25°C and 60% R.H. and finally 12 hours at 4°C
- second program under UV/Vis light and 5 sections; 10 grams of MXD gel 3.5% w/w (example A) in a transparent glass container for 12 hours at 4°C; 12 hours at 25°C and 60% of R.H.; 12 hours at 40°C and 60% R.H.; 12 hours at 25°C and 60% R.H. and finally 12 hours at 4°C. Every 24 hours sample aliquots were taken and treated like in the first study.

Finally, in the third study 100 g of MXD 3.5% w/w gel formulation (formulation A) were prepared in the presence of water with preservatives (nipagin, 75 mg and nipasol 25 mg in 100 ml of water) and 50 g of the starting formulation were transferred in a polypropylene container and the remaining amount in a glass container both equipped with a cap. The two containers were inserted in the climate room for three months at 25°C at 60% RH in the absence of light. Every 15 days sample fractions were taken and analyzed as indicated in the first study.

RESULTS

The results obtained in the first study are reported in tables 2 and 3.

As it may be inferred from the tables, Minoxidil is stable in the gel for 5 weeks both in the presence and in the absence of light.

Probably, the formation of the complex between Minoxidil and cyclodextrin, enables to enhance the solubility of the active principle and to reduce its light instability. Further, the slight increase of Minoxidil concentration occurring in both the studies at week 4-5, may be associated with a time-dependent water loss by evaporation from the formulation, requiring the use of a container with a seal plug especially if the product is stored for more than one month. A slight amber coloration is detected in both the formulations examined at week 5.

Table 2. Chemical stability of Minoxidil (MXD) under real conditions not protected from the light

| Time (week) | Concentration (mg/ml) | MXD% |
|---|---|---|
| 0 | 0.036 | 100 |

(continued)

| Time (week) | Concentration (mg/ml) | MXD% |
|---|---|---|
| 1 | 0.037 | 102 |
| 2 | 0.038 | 106 |
| 3 | 0.037 | 104 |
| 4 | 0.037 | 104 |
| 5 | 0.042 | 116 |

Table 3. Chemical stability of Minoxidil (MXD) under real conditions protected from the light

| Time (week) | Concentration (mg/ml) | MXD % |
|---|---|---|
| 0 | 0.036 | 100 |
| 1 | 0.036 | 100 |
| 2 | 0.035 | 98.6 |
| 3 | 0.035 | 98 |
| 4 | 0.040 | 111 |
| 5 | 0.041 | 114 |

Results from the second study showed the chemical stability of Minoxidil into the gel also after a cold-heat shock in climate room. Under such stress also the solubility of Minoxidil into the gel was not compromised, in fact the low temperature achieved in the climate room (4°C) does not result in any precipitate formation into the gel. Finally, this study confirmed the good physical stability properties of the gel (no organoleptic variation) apart from a slight amber coloration detected at the end of the study.

At last the confirmation of the chemical stability of Minoxidil was obtained also from the third study as showed for illustrative purposes in Table 4.

Table 4. Chemical stability of Minoxidil (MXD) into the gel in a polypropylene container during the study in climate room for 3 months at 25°C and RH 60%

| Time (week) | Concentration (mg/ml) | MXD % |
|---|---|---|
| 0 | 0.035 | 100 |
| 2 | 0.036 | 103 |
| 4 | 0.034 | 97 |
| 6 | 0.036 | 103 |
| 8 | 0.037 | 106 |
| 10 | 0.035 | 100 |
| 12 | 0.036 | 103 |

With regard to physical stability of the formulation it is possible to detect only a slight amber coloration of the product after three months.

EXAMPLE 10: *Comparison with the minoxidil based formulations employing hydrophilic polymers to solubilize the active principle*

To demonstrate the solubilization effect of minoxidil achieved by adopting the complexation strategy, rather than the use of hydrophilic polymers (as in US 2011/0112125) the following experimental tests were set up.

Minoxidil formulations were prepared in a total amount of 20 g each, comprising the following ingredients (see Example 4 of US 2011/0112125):

- Minoxidil 0.8 g (4 parts by weight)
- Polyvinylpyrrolidone (K90) 0.6 g (3 parts by weight)
- Water 18 g (90 parts by weight)
- 5M $H_2SO_4$ to set up pH at 3.0

Preparation method

0.8 g of minoxidil were dispersed into water under continuous stirring for 10 minutes. Subsequently 5M $H_2SO_4$ to dissolve minoxidil; pH value is 3.1.

0.6 g of polyvinylpyrrolidone were dispersed into the solution and mixed for additional 10 minutes until a complete solubilization of the polymer is obtained.

Finally, 5M NaOH was added to restore the pH value between 4-4.5. In this environment an abundant whit precipitate was observed.

Parallely, minoxidil formulations containing β-CD as hydrophilic polymer, minoxidil and water were prepared according to Example 3 of US 2011/0112125, leading to analogous results.

On the basis of these experimental tests, it may be inferred that the solubilization strategy adopted in the patent application US 2011/0112125 is minoxidil salification induced by the acid environment created by $H_2SO_4$ addition, being the minoxidil pKa equal to 4.6.

However, when pH is restored to pH values greater than 3 by adding NaOH, minoxidil unprotonated form reappears leading to the precipitation of the product.

In fact, to demonstrate that the precipitated product was minoxidil, the recovered precipitate from the tests was analyzed by NMR, Gas chromatography mass and FT-IR. The results of the analysis confirmed that the recovered product was minoxidil.

Finally, to confirm that the strategy underlying the formulations according to the invention is the complexation and that the pH change does not affect the active principle solubilization, the same preceding test was repeated but with the following formulation:

|  |  |
|---|---|
| - Minoxidil | 0.5 g (3.7 parts by weight) |
| - HP-β-CD (0.64) | 3.3 g (24.5 parts by weight) |
| - Water | 9 g (67 parts by weight) |
| - 5M $H_2SO_4$ q.s. to setpHat 3.0 | |

(total volume 0.650 ml)

HP-β-CD was solubilized into 9 g water under continuous stirring for 30 minutes; 0.5 g of minoxidil were added to the solution and solubilized, under magnetic stirring, for 15 minutes. pH value of this solution is equal to 7.7.

Subsequently, 5M $H_2SO_4$ was added until a pH value of 2.3 was reached.

At last, 5M NaOH was added to restore the pH value between 4 and 4.5.

Surprisingly such pH variations did not result in the formation of any precipitate.

BIBLIOGRAPHY

- US 4,596,812
- US 4,139,619
- Brusa P. and Antonio Germano. Prontuario pratico di galenica. Editor Ambrosiana. September 2011.
- Messenger AG, Rundegren J. Br. J Dermatol. 2004; 150:186-194.
- Tata S, Weiner N, Flynn G. J. Pharm Sci 1994; 19 Ott; 83(10):1508-10.
- Tata S, Flynn GL, Weiner ND. J Pharm Sci.1995 Jun; 84(6):688-691.
- Edward S, Friedman BS, Friedman PM, Cohen DE Washenic K. J. Am. Acad. Dermatol. 2002; 46:309-312.
- Balakrishnan P, Shanmugam S, Lee WS, Lee WM, Kim JO Oh DH Kim JS Yoo BK, Choi HG, Woo JS, Yong CS. Int. J. Pharm. 2009; 377:1-8.
- WO95/25500
- US 2011/0112125
- Mura S, Manconi M, Sinico C, Valenti D, Fadda AM. Int. J. Pharm 2009; 380:72-79.
- Mura S, Manconi M, Valenti D, Sinico C, Amparo OV, Fadda AM. J.D. Targ. 2011; 19(3): 189-196.
- Zhao Y, Brown MB, Jones SA. Int J Pharm 2010; 383:277-284.

- Mura S, Pirot F, Manconi M, Falson F, Fadda AM. J. Drug Target 2007; 15:101-108.
- EP 0177223
- US 5,030,442
- US 4,828,837
- Stella VJ, Rajewski RA. Pharm Res 1997; 14:556-567.
- Calderini A Pessine FBT. J Incl Phenom macrocycl Chem 2008; 60: 369-377.
- Kim J, Lee M, Rang MJ. Drug Deliv. 2003; 10(2): 119-123.
- Teak Kwan Kwon, Jin Chul Kim International Journal of Pharmaceutics 2010; 392: 268-273.
- Lopedota A, Cutrignelli A, Denora N , Laquintana V, Lopalco A, Selva S, Ragni L, Tongiani S, Franco M. Drug Development and Industrial Pharmacy. Marzo 2014.
- Irie T e Uekama K. Journal of Pharmaceutical Sciences 1997 Vol. 86, No. 2, Febbraio; pagine 147-162.
- Duchene D., Wouessidjewe D., Poelman M.-C. In New Trends in Cyclodextrins and Derivatives; Duchene, D., Ed.; Editions de Sant^mParis, 1991; pagine 447-481.
- Higuchi T, Connors KA. (1965). Phase solubility techniques. Adv Anal Chem Instrum 4:117-212.

## Claims

1. A viscose solution or gel formulation for topical treatment of androgenetic alopecia comprising:

    - minoxidil as active principle in a concentration comprised between 0.5% and 6% w/w;
    - hydroxypropyl β-cyclodextrin as solubilizing agent in a concentration comprised between 5% and 45% w/w, wherein the substitution degree of the hydroxypropyl β-cyclodextrin is 4.5;
    - at least an hydrophilic polymer selected among alginate, carbomer, polyacrylate, cellulose derivate, gelatine and pectine in a concentration comprised between 0.2% and 3% w/w;
    - water between 42% and 85% w/w;
    optionally together with one or more pharmaceutically acceptable excipients and/or adjuvants, **characterized by** the absence of the cosolvents ethanol and propylene glycol.

2. Formulation according to claim 1, further comprising at least a gelling agent selected between inorganic and organic strong bases or chlorides containing bivalent ions in a concentration comprised between 2.6% w/w and 4% w/w.

3. Formulation according to any one of the claims 1-2, wherein said hydrophilic polymer is present in a percentage comprised between 0.2% and 2%.

4. Formulation according to any one of the claims 1-3, comprising an additional β-cyclodextrin selected between sulfobutyl ether β-cyclodextrin and methyl β-cyclodextrin with variable substitution degree in combination with hydroxypropyl β-cyclodextrin, in a percentage comprised between 5% and 45% w/w.

5. Formulation according to any one of the claims 1-4, wherein the optional excipients are hydrophilic excipients selected from the group consisting in polyoxyethyleneglycol, polyvinylalcohol, polyvinyl pirrolidone, sorbitol, glycerine, lactose, saccharose, mannitol, in a concentration comprised between 0.2% and 5%.

6. Formulation according to any one of the claims 1-5, further comprising preservative agents such as antimicrobial and/or antifungal agents.

7. Formulation according to any one of the claims 1-6, wherein said at least one hydrophilic polymer is selected between sodium alginate, carbomer and polyacrylate.

## Patentansprüche

1. Viskose Lösung oder Gelformulierung zur topischen Behandlung androgenetischer Alopezie, umfassend:

    - Minoxidil als Wirkstoff in einer Konzentration zwischen 0,5 und 6% Gew.-%;
    - Hydroxypropyl-β-cyclodextrin als Lösungsvermittler in einer Konzentration zwischen 5 und 45% Gew.-%, wobei der Substitutionsgrad des Hydroxypropyl-β-cyclodextrins 4,5 beträgt;
    - wenigstens ein hydrophiles Polymer, ausgewählt unter Alginat, Carbomer, Polyacrylat, Cellulosederivat, Gelatine und Pektin, in einer Konzentration zwischen 0,2 und 3 Gew.-%;
- Wasser zwischen 42 und 85% Gew.-%;
gegebenenfalls zusammen mit einem oder mehreren pharmazeutisch verträglichen Exzipienten und/oder Adjuvantien,
**gekennzeichnet durch** die Abwesenheit der Co-Lösungsmittel Ethanol und Propylenglykol.

2. Formulierung nach Anspruch 1, außerdem umfassend wenigstens ein Geliermittel, ausgewählt unter anorganischen und organischen starken Basen oder Chloriden, die zweiwertige Ionen in einer Konzentration zwischen 2,6 Gew.-% und 4 Gew.-% enthalten.

3. Formulierung nach einem der Ansprüche 1-2, wobei das hydrophile Polymer in einem prozentualen Anteil zwischen 0,2% und 2% vorhanden ist.

4. Formulierung nach einem der Ansprüche 1-3, umfassend ein zusätzliches β-Cyclodextrin, ausgewählt unter Sulfobutylether-β-cyclodextrin und Methyl-β-cyclodextrin mit variablem Substitutionsgrad in Kombination mit Hydroxypropyl-β-cyclodextrin, in einem prozentualen Anteil zwischen 5 und 45% Gew.-%.

5. Formulierung nach einem der Ansprüche 1-4, wobei die optionalen Exzipienten hydrophile Exzipienten in einer Konzentration zwischen 0,2% und 5% sind, welche ausgewählt sind unter Polyoxyethylenglykol, Polyvinylalkohol, Polyvinylpyrrolidon, Sorbitol, Glycerin, Lactose, Saccharose, Mannit.

6. Formulierung nach einem der Ansprüche 1-5, außerdem umfassend Konservierungsmittel, wie antimikrobielle und/oder antimykotische Mittel.

7. Formulierung nach einem der Ansprüche 1-6, wobei das wenigstens eine hydrophile Polymer unter Natriumalginat, Carbomer und Polyacrylat ausgewählt ist.

## Revendications

1. Solution de viscose ou formulation de gel pour le traitement topique de l'alopécie androgénétique comprenant :

   - du minoxidil en tant que principe actif en une concentration comprise entre 0,5 % et 6 % en p/p ;
   - de l'hydroxypropyl β-cyclodextrine en tant qu'agent solubilisant en une concentration comprise entre 5 % et 45 % en p/p, dans laquelle le degré de substitution de l'hydroxypropyl β-cyclodextrine est de 4,5 ;
   - au moins un polymère hydrophile choisi parmi un alginate, un carbomère, un polyacrylate, un dérivé de cellulose, une gélatine et une pectine en une concentration comprise entre 0,2 % et 3 % en p/p ;
   - de l'eau entre 42 % et 85 % en p/p ;
   optionnellement conjointement avec un ou plusieurs excipients et/ou adjuvants pharmaceutiquement acceptables,
   **caractérisée par** l'absence des cosolvants éthanol et propylène glycol.

2. Formulation selon la revendication 1, comprenant en outre au moins un agent gélifiant choisi parmi des bases fortes inorganiques et organiques ou des chlorures contenant des ions bivalents en une concentration comprise entre 2,6 % en p/p et 4 % en p/p.

3. Formulation selon l'une quelconque des revendications 1 à 2, dans laquelle ledit polymère hydrophile est présent en un pourcentage compris entre 0,2 % et 2 %.

4. Formulation selon l'une quelconque des revendications 1 à 3, comprenant une β-cyclodextrine additionnelle choisie parmi la sulfobutyl éther β-cyclodextrine et la méthyl β-cyclodextrine ayant un degré de substitution variable en combinaison avec de l'hydroxypropyl β-cyclodextrine, en un pourcentage compris entre 5 % et 45 % en p/p.

5. Formulation selon l'une quelconque des revendications 1 à 4, dans laquelle les excipients optionnels sont des excipients hydrophiles choisis dans le groupe constitué du polyoxyéthylèneglycol, du poly(alcool vinylique), de la polyvinyl pyrrolidone, du sorbitol, de la glycérine, du lactose, du saccharose, du mannitol, en une concentration comprise entre 0,2 % et 5 %.

**6.** Formulation selon l'une quelconque des revendications 1 à 5, comprenant en outre des agents de conservation tels que des agents antimicrobiens et/ou antifongiques.

**7.** Formulation selon l'une quelconque des revendications 1 à 6, dans laquelle ledit au moins un polymère hydrophile est choisi parmi un alginate de sodium, un carbomère et un polyacrylate.

FIG.1

FIG.2

FIG.3

A      B      C

FIG.4

GROUP I

GROUP II

GROUP III

FIG.5

GROUP I

GROUP II

GROUP III

FIG.6

GROUP I

GROUP II

GROUP III

FIG.7

GROUP I

GROUP II

GROUP III

FIG.8

GROUP III          GROUP II          GROUP I

## FIG.9

Week of treatment

Legend: rat Gel.1, rat Gel.1, rat Solution.1, rat Solution.2, Ctrl.1, Ctrl.2

## FIG.10

FIG.11

*Bnip3*

A

*Atrogin1*

B

FIG.12

FIG.13

FIG.14

Phase solubility diagram MXD/HPβCD at 25°C

FIG.15

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4596812 A
- US 4139619 A
- WO 9525500 A
- US 20110112125 A
- EP 0177223 A
- US 5030442 A
- US 4828837 A
- US 20110112125 A1

### Non-patent literature cited in the description

- **BRUSA P. ; ANTONIO GERMANO.** *Prontuario pratico di galenica,* September 2011
- **MESSENGER AG ; RUNDEGREN J. BR.** *J Dermatol.,* 2004, vol. 150, 186-194
- **TATA S ; WEINER N ; FLYNN G.** *J. Pharm Sci,* 1994, vol. 83 (10), 1508-10
- **TATA S ; FLYNN GL ; WEINER ND.** *J Pharm Sci.,* June 1995, vol. 84 (6), 688-691
- **EDWARD S ; FRIEDMAN BS ; FRIEDMAN PM ; COHEN DE ; WASHENIC K.** *J. Am. Acad. Dermatol.,* 2002, vol. 46, 309-312
- **BALAKRISHNAN P ; SHANMUGAM S ; LEE WS ; LEE WM ; KIM JO OH DH KIM JS YOO BK ; CHOI HG ; WOO JS ; YONG CS.** *Int. J. Pharm.,* 2009, vol. 377, 1-8
- **MURA S ; MANCONI M ; SINICO C ; VALENTI D ; FADDA AM.** *Int. J. Pharm,* 2009, vol. 380, 72-79
- **MURA S ; MANCONI M ; VALENTI D ; SINICO C ; AMPARO OV ; FADDA AM.** *J.D. Targ.,* 2011, vol. 19 (3), 189-196
- **ZHAO Y ; BROWN MB ; JONES SA.** *Int J Pharm,* 2010, vol. 383, 277-284
- **MURA S ; PIROT F ; MANCONI M ; FALSON F ; FADDA AM.** *J. Drug Target,* 2007, vol. 15, 101-108
- **STELLA VJ ; RAJEWSKI RA.** *Pharm Res,* 1997, vol. 14, 556-567
- **CALDERINI A ; PESSINE FBT.** *J Incl Phenom macrocycl Chem,* 2008, vol. 60, 369-377
- **KIM J ; LEE M ; RANG MJ.** *Drug Deliv.,* 2003, vol. 10 (2), 119-123
- **TEAK KWAN KWON ; JIN CHUL KIM.** *International Journal of Pharmaceutics,* 2010, vol. 392, 268-273
- **LOPEDOTA A ; CUTRIGNELLI A ; DENORA N ; LAQUINTANA V ; LOPALCO A ; SELVA S ; RAGNI L ; TONGIANI S ; FRANCO M.** *Drug Development and Industrial Pharmacy,* March 2014
- **IRIE T E ; UEKAMA K.** *Journal of Pharmaceutical Sciences,* February 1997, vol. 86 (2), 147-162
- **DUCHENE D., WOUESSIDJEWE D., POELMAN M.-C.** *New Trends in Cyclodextrins and Derivatives,* 1991, 447-481
- **HIGUCHI T ; CONNORS KA.** Phase solubility techniques. *Adv Anal Chem Instrum,* 1965, vol. 4, 117-212